# EUROPEAN PATENT SPECIFICATION

(11) **EP 0 431 561 B1**
(45) Date of publication and mention of the grant of the patent: **01.02.1995**
(21) Application number: 90123219.9
(22) Date of filing: 04.12.1990
(51) Int. Cl.: C07C 231/12, C07C 235/60

(54) **Pyridinium hydrochloride metoclopramide demethylation**
Demethylierung von Metoclopramid mit Pyridiniumhydrochlorid
Déméthylation du métoclopramide à l'aide du chlorure de pyridinium

(30) Priority: 05.12.1989 US 446074
(43) Date of publication of application: 12.06.1991
(73) Proprietor: MARION MERRELL DOW INC., Kansas City Missouri 64114-3321 (US)
(72) Inventor: Wynberg, Hans, NL-9752 PA Haren (NL); Van Echten, Erik, NL-9407 RN Assen (NL); Hoeve, Wolter ten, NL-9761 VH Elde (NL)
(74) Representative: VOSSIUS & PARTNER

(56) References cited:
- US-A- 4 820 715
- L.F. FIESER et al.: "Reagents for Organic Synthesis", 1967, pages 964-966, John Wiley and Sons, New York, US

## Description

This invention concerns demethylation of metoclopramide, or suitable salt thereof, useful for providing a useful pharmaceutical or intermediate.

Various quinuclidinyl benzamide drugs, e.g., 2-[(2-methylsulfinyl)ethoxy]-4-amino-5-chloro-N-[(2-diethylamino)ethyl]benzamide, to include its pharmaceutically acceptable salt(s), e.g., its hydrochloride (ML-1035), especially useful for treatment of reflux esophagitis (heartburn), are known; see e.g., US-A-4,820,715.

Metoclopramide, i.e., 2-methoxy-4-amino-5-chloro-N-[(2-diethylamino)ethyl]benzamide, is a well-known, commercially available antiemetic compound; see e.g., US-A-3,177,252.

Metoclopramide is known to be demethylated by various procedures, providing 2-hydroxy-4-amino-5-chloro-N-[(2-diethylamino)ethyl]benzamide, an intermediate to such benzamide drugs; see e.g., US-A-4,820,715, column 11, line 56, to column 12, line 18.

Fieser & Fieser, "Reagents for Organic Synthesis," John Wiley and Sons, Inc., New York (1967), at pages 964-6, reports on pyridine (pyridinium) hydrochloride. It reports that the reagent is useful for cleavage of phenol methyl ethers. It also reports that N-alkylcarboxylic acid amides and anilides are dealkylated in good yield when heated with an excess of the reagent at 190-200°C.

The art lacks and needs improved procedures to prepare quinuclidinyl benzamide drugs, particularly through the hydroxybenzamide intermediate thereof.

Provided is a procedure for preparing 2-hydroxy-4-amino-5-chloro-N-[(2-diethylamino)ethyl]benzamide comprising contacting metoclopramide, or suitable salt thereof, with pyridinium hydrochloride.

Prepared is a useful benzamide drug intermediate.

Notably, this invention fulfills lacks and needs in the art, to include the aforesaid, and it is simple and efficient. Yields of product hydroxybenzamide can be excellent, and surprisingly so, thus at least about 80 or even at least about 90 percent of theory.

Herein, a procedure is a method and/or process.

The 2-hydroxy-4-amino-5-chloro-N-[(2-diethylamino)ethyl]benzamide is prepared by contacting metoclopramide, or suitable salt thereof, with pyridinium hydrochloride. Conditions in general are those sufficient to prepare said hydroxybenzamide.

The metoclopramide, or suitable salt thereof, can be obtained or prepared by known procedures; see e.g., US-A-3,177,252.

The pyridinium hydrochloride can be obtained or prepared by known procedures. See e.g., Fieser & Fieser, supra. The pyridinium hydrochloride can be prepared in situ.

Amounts of reagents or other substances employed can vary. Typically, molar ratios of the metoclopramide, or suitable salt thereof, to the pyridinium hydrochloride are from 1:10 to 1:2.

The metoclopramide, or suitable salt thereof, can be contacted neat with the pyridinium hydrochloride. Alternatively, a suitable diluent may be employed. In any event, a mixture generally results.

The mixture is generally heated to provide more efficient production of product hydroxybenzamide. Typically, heating temperatures are from 150 to 225°C.

The heating is carried out for a suitable time. Typically, times of heating are about from 1/2 to 5 hours.

An inert atmosphere can be employed. Inert atmospheres include such gases as nitrogen, helium and argon.

The product hydroxybenzamide can be conveniently isolated by contacting hot mixture, which may solidify otherwise if cooled to about from 100 to 140°C, particularly if derived from neat starting material, with a suitable, e.g., alkali metal, base. The base is typically cool, to include a temperature about room temperature.

Neutralization with a suitable acid generally can precipitate the hydroxybenzamide product. The suitable acid is desirably selected from mineral acids.

The hydroxybenzamide product can be recovered by known methods. Thus, for example, filtering, decanting, scooping, and so forth, may be so employed.

The hydroxybenzamide product can be used to prepare the quinuclidinyl benzamide drugs., e.g., ML-1035, by known methods. See e.g., Monkovic et al., supra.

The following further illustrates this invention.

### Example

Pyridine (800 mL) and concentrated hydrochloric acid (880 mL) were mixed in a 3-L flask. The mixture was heated, and water was distilled off until the internal temperature reached 210°C. A total of about 700 mL water was collected. The resultant pyridinium hydrochloride was allowed to cool to about 140°C. Then, 672 g of metoclopramide monohydrochloride (2.0 mol) was added. The mixture was heated at 185-190°C internal temperature for 2 hours under nitrogen. The liquid was allowed to cool somewhat and was poured into 2.5 L of a 4N aqueous sodium hydroxide solution with mechanical stirring. Another 500 mL of 4N NaOH (aq.) was added. The clear solution obtained was extracted twice with separate 500 mL portions of toluene. The aqueous layer was neutralized with concentrated hydrochloric acid (about 350 mL). The precipitate was collected, washed with water and dried under vacuum at about 40°C to yield 527.5 g of 2-hydroxy-4-amino-5-chloro-N-[(2-diethylamino)ethyl]benzamide (93 percent yield of theory).

## Claims

1. A procedure for preparing 2-hydroxy-4-amino-5-chloro-N-[(2-diethylamino)ethyl]benzamide comprising contacting metoclopramide, or suitable salt thereof, with pyridinium hydrochloride.

2. The procedure of claim 1, further comprising isolating said hydroxybenzamide with a suitable base.

3. The procedure of claim 2, further comprising neutralization with a suitable acid.

4. A procedure according to claim 1 for preparing 2-hydroxy-4-amino-5-chloro-N-[(2-diethylamino)ethyl]benzamide comprising contacting, substantially neat, a hydrochloride salt of metoclopramide with pyridinium hydrochloride in a molar ratio of the hydrochloride from 1:10 to 1:2 to provide a mixture, heating the mixture at a temperature from 150 to 225°C, isolating product hydroxybenzamide by employing an alkali metal base, neutralizing with a mineral acid, and recovering the 2-hydroxy-4-amino-5-chloro-N-[(2-diethylamino)ethyl]benzamide as a solid.

5. The procedure of any one of claims 2 to 4, wherein sodium hydroxide and hydrochloric acid are employed as said base and acid.

## Patentansprüche

1. Verfahren zur Herstellung von 2-Hydroxy-4-amino-5-chloro-N-[(2-diethylamino)ethyl]benzamid, das das Zusammenbringen von Metoclopramid oder einem geeigneten Salz davon mit Pyridiniumhydrochlorid umfaßt.

2. Verfahren nach Anspruch 1, das ferner das Isolieren des Hydroxybenzamids mit einer geeigneten Base umfaßt.

3. Verfahren nach Anspruch 2, das ferner die Neutralisation mit einer geeigneten Säure umfaßt.

4. Verfahren nach Anspruch 1 zur Herstellung von 2-Hydroxy-4-amino-5-chlor-N-[(2-diethylamino)ethyl]benzamid, umfassend das Zusammenbringen, im wesentlichen unverdünnt, von einem Hydrochloridsalz von Metoclopramid mit Pyridiniumhydrochlorid in einem Molverhältnis des Hydrochlorids von 1:10 bis 1:2, wobei ein Gemisch bereitgestellt wird, Erhitzen des Gemischs bei einer Temperatur von 150 bis 225°C, Isolieren des Hydroxybenzamidprodukts durch Einsetzen einer Alkalimetallbase, Neutralisieren mit einer Mineralsäure und Abtrennen des 2-Hydroxy-4-amino-5-chlor-N-[(2-diethylamino)ethyl]benzamids als Feststoff.

5. Verfahren nach einem der Ansprüche 2 bis 4, wobei Natriumhydroxid und Salzsäure als Base und Säure eingesetzt werden.

## Revendications

1. Procédé de préparation de 2-hydroxy-4-amino-5-chloro-N-[(2-diéthylamino)éthyl]benzamide comprenant la mise en contact du métoclopramide, ou de son sel adapté, avec du chlorhydrate de pyridinium.

2. Procédé selon la revendication 1, comprenant de plus l'isolement dudit hydroxybenzamide avec une base adaptée.

3. Procédé selon la revendication 2, comprenant de plus une neutralisation avec un acide adapté.

4. Procédé selon la revendication 1 de préparation du 2-hydroxy-4-amino-5-chloro-N-[(2-diéthylamino)éthyl]benzamide comprenant la mise en contact, pratiquement de manière non diluée, d'un sel, du type chlorhydrate, de métoclopramide avec du chlorhydrate de pyridinium en un rapport molaire du chlorhydrate compris entre 1:10 et 1:2 pour obtenir un mélange, le chauffage du mélange à une température comprise entre 150 et 225°C, l'isolement du produit hydroxybenzamide en utilisant une base de type métal alcalin, la neutralisation avec un acide minéral et la récupération du 2-hydroxy-4-amino-5-chloro-N-[(2-diéthylamino)éthyl]benzamide sous forme de solide.

5. Procédé selon l'une quelconque des revendications 2 à 4, dans lequel on utilise l'hydroxyde de sodium et l'acide chlorhydrique pour ladite base et ledit acide.
